# FASCICULE DE BREVET EUROPEEN

(11) **EP 3 522 800 B1**
(45) Date de publication et mention de la délivrance du brevet: **14.07.2021**
(21) Numéro de dépôt: 17780721.1
(22) Date de dépôt: 05.10.2017
(51) Int. Cl.: A61B 17/3207, A61F 2/24, A61B 17/34, A61N 1/05, A61B 18/16, A61N 1/36

(54) **ENSEMBLE DE REMPLACEMENT D'UNE VALVE CARDIAQUE OU D'UN ENSEMBLE D'ANGIOPLASTIE CORONAIRE**
ANORDNUNG ZUM ERSETZEN EINER HERZKLAPPE ODER EINER KORONARANGIOPLASTIEANORDNUNG
ASSEMBLY FOR REPLACING A HEART VALVE OR A CORONARY ANGIOPLASTY ASSEMBLY

(30) Priorité: 07.10.2016 FR 1659669; 07.10.2016 FR 1659671; 07.10.2016 FR 1659673
(43) Date de publication de la demande: 14.08.2019
(73) Titulaire: Electroducer, 38240 Meylan (FR)
(72) Inventeur: FAURIE, Benjamin, 38240 Meylan (FR)
(74) Mandataire: Nony
(86) Numéro de dépôt international: PCT/EP2017/075362
(87) Numéro de publication internationale: WO 2018/065523

(56) Documents cités:
- WO-A1-95/20360
- US-A- 3 893 461
- US-A- 4 541 440
- US-A- 5 733 323
- US-A1- 2002 198 583
- US-A1- 2006 241 704
- US-A1- 2009 270 941
- US-A1- 2009 318 943
- US-A1- 2010 010 551
- US-A1- 2011 251 683
- US-A1- 2012 130 220
- US-A1- 2013 096 555
- US-A1- 2013 178 908

## Description

### Domaine technique

La présente invention concerne un ensemble de remplacement d'une valve cardiaque par voie percutanée ou un ensemble d'angioplastie coronaire, comprenant un cathéter de délivrance de valve et le cas échéant un dispositif d'introduction, couramment appelé « introducteur ».

La présente invention a trait plus particulièrement à l'amélioration de l'assistance au remplacement par sidération cardiaque au moyen d'un stimulateur cardiaque.

Bien que décrit en référence au remplacement d'une valve aortique, l'ensemble selon l'invention peut être utilisé en tant qu'ensemble d'angioplastie coronaire nécessitant ou non la pose d'une prothèse couramment appelée « stent », en particulier en situation d'urgence ou même encore dans des procédures d'intervention complexe.

De même, bien que décrit en référence au remplacement d'une valve aortique, l'ensemble selon l'invention peut tout aussi bien s'appliquer pour le remplacement d'une autre valve du cœur, telle que la valve triscupide ou la valve mitrale.

De manière générale, l'introducteur et/ou le cathéter de délivrance de l'ensemble selon l'invention peut être implanté par voie percutanée au sein d'un patient, et plus précisément, par voie trans-fémorale, par voie trans-aortique, par voie carotidienne ou par voie dite sous-clavière.

### Etat de la technique

Une maladie cardiaque largement connue est celle liée au rétrécissement par calcification de la valve triscupide ou de la valve aortique cardiaque, cette dernière étant la valve qui sépare une cavité cardiaque, à savoir le ventricule gauche de l'aorte et qui permet en position ouverte de laisser passer le sang du cœur vers le reste de l'organisme d'un être humain.

Un rétrécissement serré ou très serré empêche la valve aortique de s'ouvrir normalement et donc génère la maladie aussi appelée sténose aortique calcifiée.

Le traitement de cette maladie consiste à remplacer la valve aortique cardiaque défectueuse.

Le remplacement d'une valve aortique cardiaque défectueuse est le plus fréquemment, réalisé par ouverture du thorax, mise du patient sous circulation extracorporelle, arrêt cardiaque temporaire, et ouverture du coeur, en vue de l'exérèse et du remplacement de la valve native par une valve artificielle ou de prothèse.

Ces étapes successives de l'opération ont pour inconvénients majeurs d'impliquer une hospitalisation relativement longue du patient, d'être complexe et coûteuse et de n'être réservée qu'à une partie des patients atteints, car dans bon nombre de cas, le(s) médecin(s) et/ou chirurgien(s) considèrent que l'intervention chirurgicale dite « à cœur ouvert » ne peut être pratiquée car trop risquée compte tenu de l'état général du patient, notamment du fait de l'arrêt du cœur nécessaire et de la circulation extracorporelle afférente.

Pour remédier à cet inconvénient, il a été envisagé de remplacer une valve cardiaque par voie peu invasive mais toujours sous circulation extracorporelle. On peut citer ici les demandes de brevet internationales WO 93/01768 et WO 97/28807, ainsi que les brevets américains US 5814097, US 5370685 ou US 5545214 qui illustrent des techniques peu invasives connues ainsi que des instruments de mise en œuvre de ces techniques.

Les techniques existantes ont toutefois été considérées comme n'étant pas parfaitement satisfaisantes et comme susceptibles d'être améliorées.

En particulier, ces techniques ont comme inconvénients majeurs :
- d'imposer en tout état de cause la mise du patient sous circulation extracorporelle; elles sont difficiles à mettre en pratique;
- de ne pas permettre un contrôle précis du diamètre selon lequel la valve native est coupée, en vue du calibrage ultérieur de la valve prothétique;
- d'entraîner des risques de diffusion de fragments de valve native, souvent calcifiée, dans l'organisme, qui peuvent conduire à une embolie,
- des risques de perforation de la paroi aortique ou cardiaque ;
- des risques de reflux aigus du sang lors de l'ablation de la valve native.

Pour pallier les insuffisances de ces techniques, une approche a été la mise en place de valves aortiques artificielles, dites percutanées qui s'inspire des techniques de traitemement endovasculaire par introduction d'un cathéter à l'intérieur d'un vaisseau sanguin, tel que l'aorte.

Ainsi, la valve aortique cardiaque native qui est déficiente par calcification est remplacée par une valve artificielle en évitant l'intervention habituelle de chirurgie cardiaque lourde comme évoqué ci-avant.

La mise en place d'une valve artificielle peut être actuellement réalisée par différentes voies percutanées : par voie trans-fémorale, c'est-à-dire par introduction depuis l'artère fémorale jusqu'au cœur ou par voie trans-apicale, ou par voie trans-aortique, ou par voie carotidienne ou encore par voie sous-clavière, c'est-à-dire toute voie ne nécessitant ni opération à cœur ouvert par l'intermédiaire d'une ouverture du thorax ni circulation extracorporelle.

L'opération en elle-même consiste à déposer une valve artificielle (prothèse), qui reproduit la forme générale d'une valve aortique native normale, au niveau de la valve aortique native calcifiée (malade), cette dernière étant laissée en place et écrasée par la prothèse.

Pour ce faire, la valve artificielle faite en péricarde, membrane fine entourant le cœur, d'origine porcine ou bovine, est fixée préalablement à l'intérieur d'un grillage métallique tubulaire extensible radialement (« stent » en anglais) et constitué par assemblage de fils en matériau à mémoire de forme, par exemple en alliage nickel-titane ou en cobalt-chrome, acier inoxydable 316L pour les stents coronaires.

Puis l'ensemble valve-grillage est comprimé à l'extrémité d'une gaine tubulaire, appelé cathéter de délivrance, qui peut être introduit soit directement dans une artère, soit à l'intérieur d'un introducteur permettant d'accéder à l'artère tout en maintenant une hémostase.

Un médecin interventionniste fait alors coulisser l'ensemble valve-grillage dans l'introducteur ou directement dans le cathéter de délivrance jusqu'à ce que ledit ensemble parvienne à la valve aortique malade. L'ensemble valve-grillage est ensuite déposé au niveau de la valve malade par dilatation d'un ballonnet avant la mise en place.

Il existe aussi des cathéters de délivrance de valve comprenant un ensemble valve-grillage sans ballonnet dans lesquels la valve est auto-expansible qui permet un dépôt de la valve qui s'expanse radialement par simple retrait de la gaine qui l'entoure et donc sans avoir à dilater un ballonnet au préalable.

On pourra se reporter pour plus de précisions aux brevets américains US 7018406, US 7892281, US8652202 et US 8747459.

Lors de la pose à proprement parler il est nécessaire d'effectuer pendant une courte période, un arrêt du cœur temporaire par stimulation ventriculaire rapide pour minimiser le flux transvalvulaire, i.e. entre valvules, et pour éviter à tout le moins réduire l'embolisation potentielle.

Cet arrêt du cœur temporaire aussi appelé couramment « sidération cardiaque » consiste ainsi à faire battre le cœur volontairement à 150 à 200 battements par minute de sorte qu'il n'y ait plus de contraction efficace, ce qui provoque une chute des pressions et simule une tachycardie ou fibrillation ventriculaire et donc une stabilisation du cœur.

Cette stabilisation du cœur permet la stabilisation du ballon et donc d'augmenter la précision de la mise en place de la valve artificielle en quelques secondes.

Il existe des cathéters de stimulation bipolaire, à deux électrodes, dits sondes d'entrainement ou de stimulation électro-systolique, pour la stimulation endocardiaque temporaire du ventricule droit.

Ces sondes de stimulation électro-systolique présentent un certain nombre d'inconvénients détaillés comme suit.

Tout d'abord, une telle sonde constitue un abord veineux central avec un risque de complication vasculaire surajouté pour la population de patients visée qui est fragile. Le registre français désigne « France 2 » qui répertorie les interventions de remplacement de valve aortique, couramment désignées sous l'acronyme anglais TAVI pour «Transcatheter Aortic Valve Implantation », a indiqué comme résultat un taux de risque de complications vasculaires importantes égal à 4,7%. Ce résultat est reporté en page 1709 de la publication [1].

Ensuite, cette sonde est relativement rigide, et de ce fait sa mise en place dans le ventricule droit qui est fragile et dont la paroi est plus fine que celle du ventricule gauche, induit un risque conséquent du phénomène bien connu des médecins interventionnistes sous le terme « tamponnade », qui traduit une insuffisance circulatoire importante pouvant aller jusqu'à la mortalité du patient.

Il est à noter d'ailleurs que ce risque existe aussi bien pendant l'intervention, c'est-à-dire lors de la mise en place de la sonde électro-systolique mais aussi en postopératoire du fait de la mobilisation des patients dans leur lit et donc de la sonde encore présente qui peut alors transpercer la paroi du ventricule droit.

De plus, il y a un risque de déplacement de la sonde de stimulation électro-systolique pendant le moment crucial de la mise en place de la valve. En effet, une sonde de stimulation n'est pas fixée dans une paroi du cœur et peut donc se déplacer et ainsi engendrer une perte de capture du signal électrique de stimulation.

Le cœur n'est alors plus stimulé et donc a des mouvements importants qui gênent le placement de la valve ou du ballon.

Un autre risque lié à l'utilisation de telles sondes est le risque d'infection au site de ponction. Le registre France 2 a indiqué un taux inférieur à 1% : voir publication [1].

Enfin, un médecin interventionniste considère comme non négligeable le temps opératoire supplémentaire lié à la mise en place d'une sonde de stimulation temporaire, qui est une opération pas toujours simple à réaliser.

La publication [2] met en avant les avantages de réaliser cette stimulation ventriculaire sur le ventricule gauche et non pas sur le ventricule droit et de le faire non pas au moyen d'un cathéter de stimulation spécifique par voie transveineuse, mais en mettant en oeuvre un stimulateur cardiaque externe avec le guide-fil utilisé pour les interventions de ce type.

Ainsi, la mise en oeuvre préconisée que l'on retrouve décrite dans cette publication [2] consiste à se servir du guide-fil supportant le ballon d'expansion du stent et introduit dans le ventricule gauche, en tant que partie reliée à la cathode d'un stimulateur cardiaque et d'une électrode cutanée ou aiguille plantée dans le tissu sous cutané, en tant que support de l'anode du stimulateur cardiaque.

Les publications [3] et [4] valident dans le cas d'une intervention par angioplastie coronaire sur une population porcine, l'efficacité d'une stimulation cardiaque temporaire avec une tension électrique de stimulation moindre, par la mise en oeuvre du guide-fil supportant le ballon d'expansion du stent, en tant que partie reliée à la cathode d'un stimulateur cardiaque et d'une électrode cutanée ou aiguille plantée dans le tissu sous cutané, en tant que support de l'anode du stimulateur cardiaque.

Ainsi, ces mises en œuvre préconisées ont pour avantages d'éviter d'avoir à implanter un cathéter supplémentaire dédié, d'éviter un accès supplémentaire au cœur, de réduire le temps et le coût de l'opération, mais aussi de diminuer le taux de complications liées à l'implantation du cathéter dédié, et ce tout en permettant une stimulation aussi efficace que par le biais d'une stimulation par voie transveineuse.

En outre, comparativement aux sondes de stimulation électro-systolique du ventricule droit qui induisent le risque de tamponnade comme explicité ci-avant, le guide-fil utilisé pour cette technique est très stable et vient en appui permanent contre la paroi du ventricule gauche relativement épaisse puisqu'il sert de rail pour avancer l'ensemble stent-ballon-valve au travers de la valve.

Cela étant, cette technique nécessite tout de même la mise en place d'une électrode ou d'une aiguille sous-cutanée supplémentaire qui doit être précise, la mise en place et le maintien de pinces de connexion, de type crocodile sur deux supports éloignés.

L'inventeur de la présente invention a déposé la demande de brevet PCT/EP2016/057385 qui décrit et revendique l'intégration d'une électrode d'un stimulateur cardiaque directement dans la gaine d'introduction (introducteur ou cathéter de délivrance) dans l'artère d'un patient. L'invention proposée permet de rendre plus aisée et plus rapide la mise en place et la manipulation des électrodes de stimulateur cardiaque pour le(s) chirurgien(s) en charge de l'opération.

L'inconvénient de cette demande est qu'elle nécessite la réalisation d'introducteurs ou de cathéters de délivrance spécifiques.

US2011/0251683 divulgue un dispositif de remplacement de valve aortique avec déploiement d'une prothèse aortique.

US 2009/0270941 divulgue un cathéter guide ou de délivrance dont la portion distale est munie d'une électrode périphérique.

US 5733323 décrit un introducteur pour cartographie cardiaque comprenant une gaine en polymère revêtue sur sa surface extérieure d'un fil mince électriquement conducteur pour former une électrode.

Or, il serait intéressant de pouvoir disposer d'une ou plusieurs solutions qui puisse(nt) être adaptable(s) aux introducteurs ou cathéters existants, c'est-à-dire ceux ne disposant pas d'électrode intégrée en leur sein.

Le but de l'invention est de répondre au moins en partie à ce besoin.

### Exposé de l'invention

Pour ce faire, l'invention a pour objet selon une première alternative un ensemble de remplacement d'une valve cardiaque par voie percutanée, comprenant :
- un dispositif formant un introducteur comprenant au moins une gaine tubulaire d'introduction, destinée à être introduite dans une artère d'un corps humain et à laisser passer un dispositif d'intervention chirurgicale, tel qu'un cathéter de délivrance,
- un manchon adapté pour être emmanché autour de la gaine d'introduction, le manchon étant en matériau électrique conducteur sur au moins une partie de sa périphérie extérieure de sorte que lorsque la gaine avec le manchon emmanché autour est introduite dans l'artère d'un corps humain, la périphérie conductrice du manchon est en contact avec le tissu sous-cutané du corps ou avec l'artère, le manchon comprenant en outre une connexion électrique à une électrode d'un stimulateur cardiaque externe au corps;
- au moins un guide-fil destiné à être introduit dans la gaine tubulaire de l'introducteur pour l'avancement d'une valve artificielle destinée à remplacer la valve cardiaque, le guide-fil comprenant une partie métallique servant en outre de connexion à l'autre électrode du stimulateur cardiaque externe.

Selon une deuxième alternative, l'invention a pour objet un ensemble de remplacement d'une valve cardiaque par voie percutanée, comprenant :
- un dispositif formant un cathéter de délivrance de valve comprenant au moins une gaine tubulaire d'introduction, destinée à être introduit dans une artère d'un corps humain,
- un manchon adapté pour être emmanché autour de la gaine d'introduction, le manchon étant en matériau électrique conducteur sur au moins une partie de sa périphérie extérieure de sorte que lorsque la gaine avec le manchon emmanché autour est introduit dans l'artère d'un corps humain, la périphérie conductrice du manchon est en contact avec le tissu sous-cutané du corps ou avec l'artère, le manchon comprenant en outre une connexion électrique à une électrode d'un stimulateur cardiaque externe au corps;
- au moins un guide-fil destiné à être introduit dans la gaine tubulaire du cathéter de délivrance pour l'avancement d'une valve artificielle destinée à remplacer la valve cardiaque, le guide-fil comprenant au moins une partie métallique servant en outre de connexion à l'autre électrode du stimulateur cardiaque externe.

Selon un mode de réalisation, l'électrode du stimulateur cardiaque connectée au manchon conducteur électrique emmanché autour de la gaine d'introduction de l'introducteur ou du cathéter de délivrance est l'anode tandis que celle connectée à la partie métallique du guide-fil est la cathode.

L'invention a également pour objet un manchon électriquement conducteur destiné à un ensemble décrit précédemment.

Le manchon peut être constitué d'une pièce monobloc en matériau conducteur, tel que du carbone.

Il peut également être constitué d'une gaine comprenant sur sa périphérie extérieure un revêtement conducteur électrique, tel qu'un revêtement en carbone.

Selon un mode de réalisation avantageux, le manchon peut être élastique de sorte à pouvoir s'emmancher sur des gaines d'introducteurs ou des cathéters de délivrance de différents diamètres, typiquement des diamètres externes compris entre 1,67 et 8 mm (entre 5 et 24 French). Typiquement, les diamètres externes peuvent valoir 4mm, 4,67mm, 5,33mm ou encore 6mm pour les gaines destinées aux ensembles de remplacement de valve cardiaque.

L'invention concerne selon une troisième alternative un ensemble de remplacement d'une valve cardiaque par voie percutanée, comprenant :
- un dispositif formant un introducteur comprenant au moins une gaine tubulaire d'introduction, destinée à être introduite dans une artère d'un corps humain et à laisser passer un dispositif d'intervention chirurgicale, tel qu'un cathéter de délivrance,
- une électrode transcutanée, comprenant une partie adhésive destinée à être collée sur la peau du corps humain dans lequel la gaine est introduite, et une partie en matériau électrique conducteur de sorte que lorsque la partie adhésive est collée à la peau, la partie conductrice peut délivrer un courant de manière transcutanée, la partie conductrice comprenant en outre une connexion électrique à une électrode d'un stimulateur cardiaque externe au corps;
- au moins un guide-fil destiné à être introduit dans la gaine tubulaire de l'introducteur pour l'avancement d'une valve artificielle destinée à remplacer la valve cardiaque, le guide-fil comprenant une partie métallique servant en outre de connexion à l'autre électrode du stimulateur cardiaque externe.

Selon une quatrième alternative, l'invention a pour objet un ensemble de remplacement d'une valve cardiaque par voie percutanée, comprenant :
- un dispositif formant un cathéter de délivrance de valve comprenant au moins une gaine tubulaire d'introduction, destinée à être introduit dans une artère d'un corps humain,
- une électrode transcutanée, comprenant une partie adhésive destinée à être collée sur la peau du corps humain dans lequel la gaine est introduite, et une partie en matériau électrique conducteur de sorte que lorsque la partie adhésive est collée à la peau, la partie conductrice peut délivrer un courant de manière transcutanée, la partie conductrice comprenant en outre une connexion électrique à une électrode d'un stimulateur cardiaque externe au corps;
- au moins un guide-fil destiné à être introduit dans la gaine tubulaire du cathéter de délivrance pour l'avancement d'une valve artificielle destinée à remplacer la valve cardiaque, le guide-fil comprenant au moins une partie métallique servant en outre de connexion à l'autre électrode du stimulateur cardiaque externe.

Selon un mode de réalisation, l'électrode du stimulateur cardiaque connectée à l'électrode transcutanée est l'anode tandis que celle connectée à la partie métallique du guide-fil est la cathode.

L'invention a également pour objet une électrode transcutanée, destinée à un ensemble décrit précédemment.

L'invention a également, selon une cinquième alternative, un ensemble de remplacement d'une valve cardiaque par voie percutanée, comprenant :
- un dispositif formant un introducteur comprenant au moins une gaine tubulaire d'introduction, destinée à être introduite dans une artère d'un corps humain et à laisser passer un dispositif d'intervention chirurgicale, tel qu'un cathéter de délivrance,
- au moins un guide-fil, dit guide-fil bipolaire, destiné à être introduit dans la gaine tubulaire de l'introducteur pour l'avancement d'une valve artificielle destinée à remplacer la valve cardiaque, le guide-fil comprenant une âme métallique revêtue d'une gaine électriquement isolante sur une portion centrale entre l'extrémité proximale et l'extrémité distale du guide-fil, l'âme métallique étant non isolée électriquement sur le reste de la longueur du guide fil, la gaine électriquement isolante intégrant un élément conducteur électrique dont une portion distale est apparente sur une au moins une partie de la périphérie extérieure de la gaine isolante de sorte à être en contact avec le tissu sous-cutané du corps ou avec l'artère et dont une portion proximale est apparente sur une au moins une partie de la périphérie extérieure de la gaine isolante de sorte à être accessible depuis l'extérieur du corps lorsque le guide-fil est introduit dans la gaine d'introduction, la portion proximale servant de connexion à une électrode d'un stimulateur cardiaque externe au corps, tandis que l'âme métallique du guide-fil bipolaire sert de connexion à l'autre électrode du stimulateur cardiaque externe.

Selon une sixième alternative, l'invention a pour objet un ensemble de remplacement d'une valve cardiaque par voie percutanée, comprenant :
- un dispositif formant un cathéter de délivrance de valve comprenant au moins une gaine tubulaire d'introduction, destinée à être introduit dans une artère d'un corps humain,
- au moins un guide-fil, dit guide-fil bipolaire, destiné à être introduit dans la gaine tubulaire d'introduction pour l'avancement d'une valve artificielle destinée à remplacer la valve cardiaque, le guide-fil comprenant une âme métallique revêtue d'une gaine électriquement isolante sur une portion centrale entre l'extrémité proximale et l'extrémité distale du guide-fil, l'âme métallique étant non isolée électriquement sur le reste de la longueur du guide fil, la gaine électriquement isolante intégrant un élément conducteur électrique dont une portion distale est apparente sur une au moins une partie de la périphérie extérieure de la gaine isolante de sorte à être en contact avec le tissu sous-cutané du corps ou avec l'artère et dont une portion proximale est apparente sur une au moins une partie de la périphérie extérieure de la gaine isolante de sorte à être accessible depuis l'extérieur du corps lorsque le guide-fil est introduit dans la gaine d'introduction, la portion proximale servant de connexion à une électrode d'un stimulateur cardiaque externe au corps, tandis que l'âme métallique du guide-fil bipolaire sert de connexion à l'autre électrode du stimulateur cardiaque externe.

Selon un mode de réalisation, l'électrode du stimulateur cardiaque connectée à l'élément conducteur électrique intégré dans la gaine du guide-fil est l'anode, tandis que celle connectée à l'âme métallique du guide-fil est la cathode.

Selon une variante avantageuse de l'invention, l'élément conducteur métallique est une couche métallique noyée dans la gaine isolante, à l'exception de ses portions apparentes, distale et proximale.

Selon un mode de réalisation, l'extrémité distale, non isolée électriquement, de l'âme métallique du guide-fil, destinée à venir en contact avec la paroi du ventricule gauche du cœur d'un patient, est une portion plus souple que le reste du guide-fil. De préférence, la portion plus souple est adaptée pour s'enrouler sur elle-même lorsqu'elle vient en contact avec la paroi du ventricule gauche du cœur d'un patient.

La portion distale de l'élément conducteur peut être reliée à une connexion électrique, destinée elle-même à être reliée à une électrode du stimulateur cardiaque externe au corps,

Également divulgué est un guide-fil bipolaire, destiné à un ensemble décrit précédemment. Un tel guide-fil bipolaire comprend une âme métallique revêtue d'une gaine électriquement isolante sur une portion centrale entre l'extrémité proximale et l'extrémité distale du guide-fil, l'âme métallique étant non isolée électriquement sur le reste de la longueur du guide fil, la gaine électriquement isolante intégrant un élément conducteur électrique dont une portion distale est apparente sur une au moins une partie de la périphérie extérieure de la gaine isolante et dont une portion proximale est également apparente sur une au moins une partie de la périphérie extérieure de la gaine isolante.

En cherchant une alternative à la solution décrite selon la demande PCT/EP2016/057385 précitée, l'inventeur a cherché parmi les solutions déjà existantes de stimulation cardiaque.

Il a alors testé une électrode de stimulation connue dédiée à la stimulation cardiaque temporaire transcutanée. Cette technique est proposée dans le traitement de la bradycardie symptomatique en situation d'urgence ou pour empêcher une tachyarythmie d'un cœur.

Mais personne n'avait pensé à tester une telle électrode en vue de réaliser une sidération cardiaque.

De manière surprenante, en reliant la cathode d'un stimulateur cardiaque externe au guide-fil d'une valve artificielle et l'anode à une électrode transcutanée en contact avec la peau du patient, l'inventeur a obtenu une sidération cardiaque efficace.

L'inventeur a également pensé à intégrer le support métallique d'une électrode du stimulateur cardiaque dans une pièce à emmanchée autour de la gaine d'introduction qui est introduite dans l'artère d'un patient. Le manchon selon l'invention est donc directement en contact avec l'artère du patient.

L'inventeur a enfin pensé à intégrer le support métallique d'une électrode du stimulateur cardiaque, non pas comme dans la demande précitée directement dans la gaine d'introduction dans l'artère d'un patient, mais dans un guide-fil.

Le guide-fil bipolaire selon l'invention comme revendiqué, sert à lui seul de support des deux connexions électriques reliées respectivement à l'anode et à la cathode d'un stimulateur cardiaque externe. Ainsi, l'âme métallique sert de support à l'une des connexions électriques tandis que la portion proximale de l'élément conducteur, isolée de l'âme métallique, sert de support à l'autre des connexions électriques.

On peut ainsi réaliser une sidération cardiaque par stimulation électrique bipolaire, ce qui a pour avantage de nécessiter un seuil électrique de stimulation très faible.

La gaine d'introduction peut être celle d'un introducteur ou directement celle d'un cathéter de délivrance, de taille réduite par rapport à celle d'un introducteur. Un cathéter de délivrance de valve ne nécessite pas de facto d'introducteur puisqu'il peut être introduit directement dans l'artère d'un patient.

Grâce aux différentes alternatives de l'invention, il n'est plus nécessaire de planter une aiguille dans les tissus sous-cutanés ou une électrode cutanée pour servir de support à l'électrode, typiquement l'anode d'un stimulateur cardiaque.

Il n'est pas non plus nécessaire d'utiliser et de mettre en place une sonde électro-systolique comme selon l'état de l'art, appelée couramment sonde temporaire.

De plus, grâce à l'invention, l'intensité de stimulation nécessaire à la sidération cardiaque est plus faible que dans les solutions selon l'état de l'art, du fait d'une impédance plus faible du système vasculaire par rapport au tissu sous-cutané. Typiquement, l'intensité du courant délivré, en vue d'une sidération cardiaque, peut aller de 10 à 20 mA et la tension délivrée de 0,5 à 10 Volt.

Le(s) chirurgien(s) en charge de l'opération peu(ven)t ainsi relier l'électrode, typiquement l'anode du stimulateur cardiaque au manchon conducteur emmanché autour de l'introducteur ou du cathéter de délivrance facilement ou encore l'électrode transcutanée ou encore le guide-fil bipolaire puis relier comme usuellement l'autre électrode, typiquement la cathode au guide-fil de l'ensemble valve-stent-ballon ou valve auto-expansible-stent.

Ainsi, l'étape de préparation à l'arrêt du cœur est plus simple et plus rapide à réaliser.

En plus, l'inventeur pense qu'un manchon ou une électrode transcutanée ou un guide-fil bipolaire selon l'invention peuvent diminuer les risques de complications liées aux sondes de stimulation électro-systolique selon l'état de l'art qui sont placées dans le ventricule droit.

L'introducteur ou le cathéter de délivrance peut être introduit comme usuellement par voie transapicale ou par voie transfémorale, qui est privilégiée pour son caractère moins invasif pour des patients plus fragiles.

L'introducteur ou le cathéter de délivrance peut intégrer un système de perfusion périphérique, usuellement appelé « Flush », qui peut être mis en place pour nettoyer l'intérieur de l'introducteur ou du cathéter de tout caillot de sang susceptible d'être présent.

Ainsi, avantageusement, pour un ensemble de remplacement de valve aortique cardiaque, l'introducteur peut être un introducteur connu, par exemple celui sous la dénomination commerciale « *ensemble de gaine d'introduction eSheath d'Edwards »* commercialisé par la société Edwards Lifesciences.

La valve artificielle peut être introduite et positionnée dans l'artère au moyen d'un cathéter de valve classique, lui-même introduit dans l'introducteur. La valve artificielle occupe alors une position repliée et ne fait pas obstacle à l'introduction et coulissement du cathéter de valve dans l'introducteur puis dans l'artère ou dans le cathéter de délivrance selon l'invention puis dans l'artère.

Puis, en position déployée, la valve artificielle vient prendre appui contre la paroi extérieure de la valve cardiaque native en lieu et place de cette dernière en l'écrasant.

Un cathéter de valve classique permet ainsi d'introduire et de positionner la valve artificielle à l'endroit adéquat, par le même geste d'opération que celui permettant d'ouvrir et d'écraser la valve native. Après ouverture et écrasement de cette dernière, le cathéter de valve est coulissé axialement dans la direction distale afin d'amener la valve artificielle au niveau adéquat dans l'ouverture de la valve native.

Le(s) chirurgien(s) intervenant sur le patient applique(nt) pendant l'ouverture et l'écrasement de la valve native puis après, une stimulation cardiaque au moyen du stimulateur cardiaque extérieur, le courant électrique circulant entre la cathode et l'anode du stimulateur, la cathode étant reliée au guide-fil de la valve artificielle et l'anode étant reliée au manchon conducteur selon l'invention, emmanché autour de la gaine tubulaire extérieure de l'introducteur ou du cathéter de délivrance et en contact avec le tissu sous-cutané du patient ou avec la paroi interne de l'artère.

Simultanément à la stimulation ventriculaire, la valve artificielle est déployée. Le cathéter de valve est ensuite retiré.

En résumé, les avantages d'un ensemble selon l'invention sont identiques à ceux d'un ensemble selon la demande PCT/EP2016/057385, que l'on peut énumérer comme suit :
- une mise en place plus simple et plus rapide d'une électrode, typiquement l'anode de stimulation ventriculaire lors de l'opération de remplacement d'une valve aortique déficiente ;
- la suppression de la nécessité de planter une aiguille sous-cutanée supplémentaire en tant que support d'électrode, typiquement d'anode de stimulateur cardiaque ;
- un temps et coût moindres de l'opération de remplacement d'une valve cardiaque déficiente ;
- une efficacité accrue de la stimulation temporaire en vue de réaliser la sidération cardiaque souhaitée du fait de la moindre impédance du système vasculaire rencontré par le courant électrique de stimulation puisque le manchon autour d'un introducteur ou d'un cathéter de délivrance est directement en contact avec ledit système, au contraire des aiguilles selon l'état de l'art qui viennent en contact avec le tissu cutané d'un patient qui présente nécessairement une impédance plus élevée;
- une efficacité accrue de la stimulation temporaire en vue de réaliser la sidération cardiaque souhaitée du fait de la stabilité du guide rigide (diamètre de l'ordre de 1,455mm) dans le ventricule gauche, au lieu de l'instabilité de la sonde électro-systolique selon l'état de l'art mise en place dans le ventricule droit ;
- la possibilité de réaliser la stimulation temporaire cardiaque avec un courant électrique moins élevé du fait de l'impédance moindre du système vasculaire rencontré entre les deux électrodes du stimulateur extérieur ;
- la suppression des risques de complication liés aux sondes de stimulation temporaires selon l'état de l'art qui sont placées dans le ventricule droit ;
- l'utilisation possible de l'introducteur ou du cathéter de délivrance pour plusieurs types d'interventions TAVI différentes, telles qu'un remplacement de valve aortique, pulmonaire, tricuspide ou mitrale. En particulier, pour le remplacement d'une valve tricuspide dégénérée, seule la technique d'introduction d'une sonde de stimulation dans le ventricule droit au moyen du guide-rail (diamètre 0,89 mm) est envisageable car il n'est pas envisageable de mettre à la fois le guide-rail et une sonde électro-systolique puisque l'expansion du ballon ou de la prothèse valvulaire viendrait comprimer la sonde avec le risque inhérent d'interruption de la stimulation ou de coincer la sonde de stimulation;
- l'utilisation possible dans la population pédiatrique lors des procédures valvulaires ou cardiaques sur des cœurs plus tachycardes, plus mobiles que dans la population adulte. De plus, il s'agit d'une population chez laquelle la ponction veineuse fémorale peut être très difficile ainsi que la mise en place d'une sonde de stimulation ventriculaire droite. Enfin, les parois ventriculaires droites infantiles sont fines et fragiles majorant ainsi le risque de complication grave telle que la tamponnade. Il s'agit d'ailleurs de la population décrite dans la publication [2] ;
- l'utilisation possible dans le domaine d'angioplastie coronaire dans l'urgence et dans les procédures complexes, dans lequel une stimulation cardiaque temporaire doit être réalisée de manière efficace et très rapidement. A cet effet, un introducteur ou un cathéter de délivrance avec un manchon selon l'invention évite le temps de mise en place d'une électrode ou d'une aiguille sous-cutanée supplémentaire comme selon l'état de l'art, ce qui peut être déterminant lors de ces interventions.

La seule relative contrainte du manchon, de l'électrode transcutanée ou du guide-fil bipolaire selon l'invention l'est au début de l'intervention lors de la préparation et consiste en un emmanchement du manchon autour de la gaine d'introduction. Mais cette opération est très simple et aisée à mettre en œuvre et peut tout-à-fait être réalisée par un(e) assistant(e) ou infirmier(ère), celui-ci (celle-ci) n'ayant pas besoin d'avoir de compétences particulières pour cette tâche.

L'invention a encore pour objet l'utilisation de l'ensemble avec introducteur ou cathéter de délivrance avec un manchon décrit précédemment pour le remplacement d'une valve aortique, pulmonaire, tricuspide ou mitrale.

L'invention a enfin pour objet l'utilisation de l'ensemble avec introducteur ou cathéter de délivrance avec un manchon décrit précédemment pour une intervention en angioplastie coronaire, en particulier en situation d'urgence.

Cette utilisation est avantageuse particulièrement dans trois situations d'interventions que l'on peut rencontrer en angioplastie coronaire.

La première est le traitement d'un infarctus aigu qui entrainerait des troubles de conduction de type bradycardie extrême ou bloc auriculo-ventriculaire de haut degré. Utiliser un introducteur avec un manchon selon l'invention permet d'éviter l'emploi d'une sonde électro-systolique selon l'état de l'art bien plus invasive et nécessitant un temps d'implantation surajouté non négligeable.

La deuxième concerne le traitement des lésions coronaires calcifiées par fraisage réalisé à l'intérieur des coronaires en question, usuellement avec un appareil de fraisage connu sous la dénomination commerciale « Rotablator® ». Utiliser un introducteur avec un manchon conducteur selon l'invention permet alors de s'affranchir également de l'utilisation d'une sonde de stimulation cardiaque électro-systolique comme selon l'état de l'art.

La troisième concerne la pose d'un stent, dans certaines parties des coronaires à proximité de leurs origines (ostia), ces zones sont très mobiles par rapport au cathéter d'intervention alors que ces zones anatomiques nécessitent une très grande précision dans l'implantation du stent. Utiliser un introducteur avec un manchon conducteur selon l'invention permet de stabiliser le stent avant et pendant sa pose.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une vue en perspective d'un introducteur selon l'état de l'art, destiné à être introduit dans une artère fémorale au niveau de l'aine d'un patient ;
- les figures 2A à 2B montrent en vue de coupe longitudinale partielle différentes étapes de coulissement d'un cathéter de valve dans l'introducteur selon la figure 1, afin de réaliser le placement d'une valve artificielle en lieu et place d'une valve aortique native déficient ;
- la figure 3 montre en vue schématique en perspective depuis l'extérieur d'un patient à la fois l'étape de mise en place d'un cathéter de valve et des électrodes de stimulation cardiaque selon l'état de l'art ;
- la figure 4 est une vue schématique en perspective d'un cathéter de délivrance selon l'état de l'art, destiné à être introduit directement dans l'artère d'un patient sans nécessité d'un introducteur ;
- la figure 5 est une vue en perspective d'un manchon électriquement conducteur selon l'invention destiné à être emmanché autour d'un dispositif formant introducteur selon l'état de l'art tel que celui représenté en figure 1 ou autour d'un cathéter de délivrance selon l'état de l'art tel que celui représente en figure 4;
- la figure 6 est une vue de coupe longitudinale partielle montrant un manchon selon la figure 5 emmanché autour d'un introducteur selon l'état de l'art, tel que celui représenté en figure 1 ;
- la figure 7 est une vue en perspective d'une électrode transcutanée destinée à être utilisée dans un ensemble de de remplacement d'une valve cardiaque par voie percutanée, afin d'obtenir une sidération cardiaque;
- la figure 8 montre en vue schématique en perspective depuis l'extérieur d'un patient à la fois l'étape de mise en place d'un cathéter de valve et des électrodes de stimulation cardiaque selon l'invention ;
- la figure 9 est une vue en perspective d'un guide-fil bipolaire selon l'invention, destiné à être utilisée dans un ensemble de de remplacement d'une valve cardiaque par voie percutanée, afin d'obtenir une sidération cardiaque;
- les figures 9A et 9B sont des vues en coupe transversale selon A-A et selon B-B, réalisées au niveau respectivement de la portion distale et proximale de l'élément électrique conducteur noyé dans la gaine isolante du guide-fil bipolaire selon la figure 9;
- la figure 10 montre en coupe la constitution de la portion centrale du guide-fil bipolaire selon l'invention ;
- la figure 11 montre en vue schématique en perspective depuis l'extérieur d'un patient à la fois l'étape de mise en place d'un cathéter de valve et des électrodes de stimulation cardiaque selon l'invention.

Dans la description qui va suivre ainsi que dans l'ensemble de la présente demande, les termes « distal » et « proximal » sont utilisés par référence avec le corps d'un patient dont la valve aortique native déficiente est remplacée par une valve aortique artificielle. Ainsi, l'extrémité distale d'un introducteur est l'extrémité située le plus à l'intérieur du patient lors de l'opération de remplacement.

Par souci de simplification, les mêmes éléments dans le dispositif selon l'invention et dans celui selon l'état de l'art sont désignés par les mêmes références.

On précise que les différents éléments ne sont pas nécessairement représentés à l'échelle.

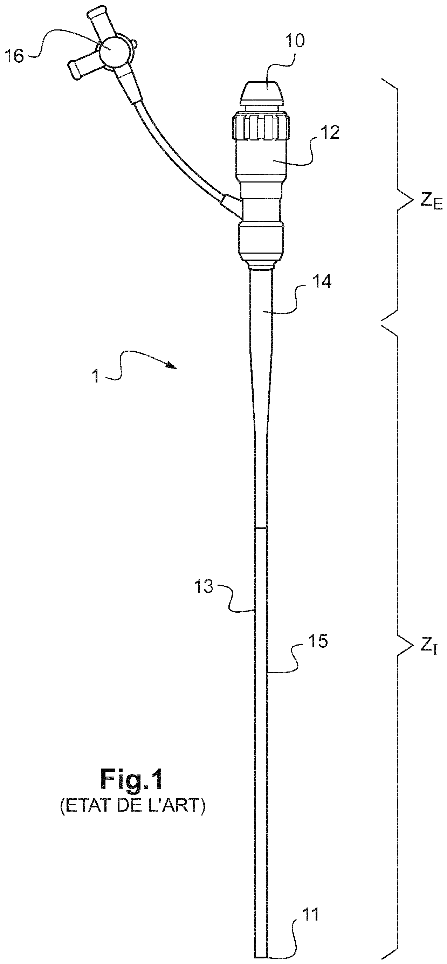

La figure 1 représente un introducteur 1 de remplacement d'une valve cardiaque par voie transfémorale.

Cet introducteur 1 de forme générale tubulaire comprend entre son extrémité proximale 10 et son extrémité distale 11, un embout 12 prolongé par au moins une gaine tubulaire 13 extérieure formée de deux portions tubulaires 14, 15 du côté proximal vers le côté distal, considérés par rapport à l'introduction dans une artère fémorale d'un patient à opérer, c'est-à-dire de haut en bas sur la figure 1.

L'embout 12 intègre en général un ensemble de vannes étanches pour réaliser une hémostase, c'est-à-dire pour assurer le maintien du sang à l'intérieur des vaisseaux sanguins du patient lors de l'intervention.

La gaine tubulaire 13 peut être extensible ou non pour pouvoir laisser passer un dispositif d'intervention chirurgicale tel qu'un cathéter de valve comme expliqué par la suite. Le matériau constitutif de la gaine 13 est un matériau biocompatible, tel qu'en silicone. Elle peut tout aussi bien être réalisée en Teflon™ ou en polyuréthane. La gaine peut être avantageusement recouverte à l'extérieur d'une couche hydrophile et à l'intérieur d'une couche à matériau à faible coefficient de frottement pour faciliter le coulissement d'un dispositif d'intervention.

L'introducteur 1 illustré en figure 1 comprend également un dispositif de rinçage 16 à robinets, couramment appelé « flush », intégré pour rincer l'intérieur de l'introducteur 1 au moyen d'un liquide de rinçage approprié.

Tous les éléments de l'introducteur 1 présents dans la zone proximale ou extérieure Z_{E} sont destinés à rester à l'extérieur du corps du patient, toute la portion distale 15 de la gaine 13 définissant la zone distale Z_{I} est destinée à être introduite dans une artère fémorale d'un patient.

L'introducteur 1 illustré est par exemple celui commercialisé sous la dénomination commerciale « *ensemble de gaine d'introduction eSheath d'Edwards »* commercialisé par la société Edwards Lifesciences.

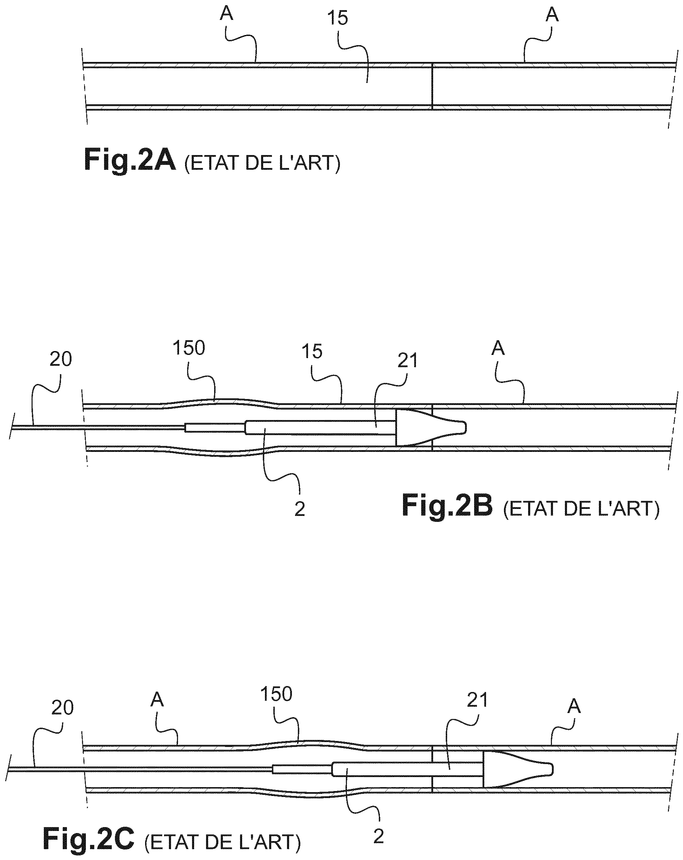

On a représenté aux figures 2A à 2C l'avancement d'un cathéter de valve 2 constitué d'un guide-fil 20 et d'un ensemble 21 constitué d'une valve artificielle fixée à un stent à expansion radiale et d'un ballon gonflable pour réaliser cette expansion, à l'intérieur de la portion distale 14 de la gaine tubulaire de l'introducteur 1 déjà introduit dans une artère fémorale A.

La pointe de l'ensemble 21 permet de pénétrer aisément la valve aortique native déficiente.

On voit sur ces figures qu'au fur et à mesure du coulissement du cathéter de valve 2, la portion 15 de la gaine tubulaire se déforme temporairement radialement en formant une légère excroissance 150. Lorsque la gaine tubulaire n'est pas extensible, elle ne se déforme pas radialement.

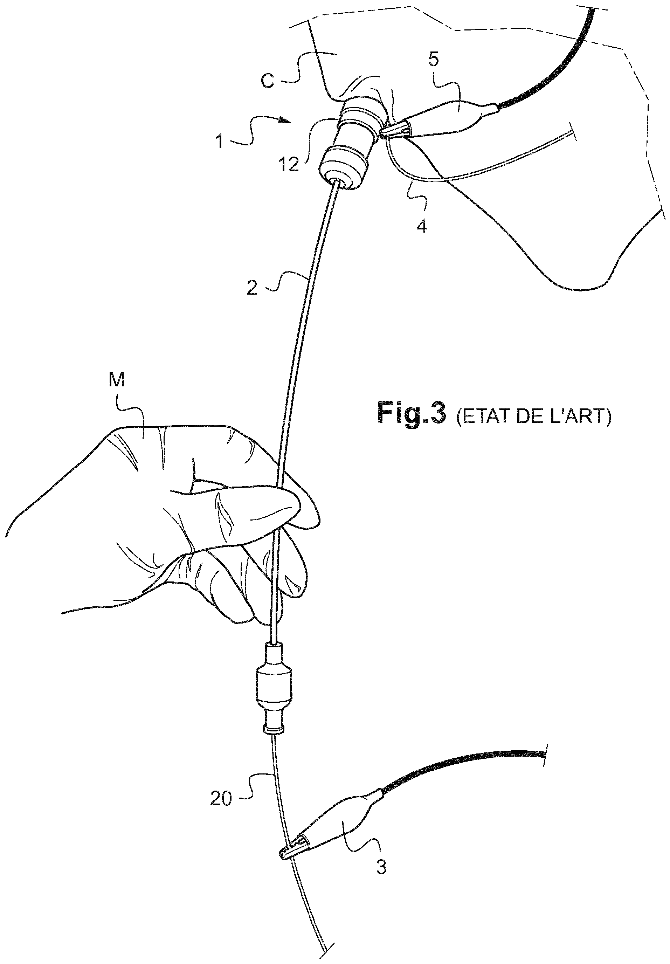

En figure 3, on peut voir que la main M d'un chirurgien réalise l'introduction du cathéter de valve 2 dans l'introducteur 1 déjà introduit dans l'artère fémorale d'un patient, l'embout 12 faisant saillie à l'extérieur du corps C.

Cette introduction du cathéter de valve 2 permet d'amener l'ensemble 21 au niveau de la valve aortique calcifiée déficiente qu'il faut remplacer.

Usuellement, comme visible également sur cette figure 3, une pince 3 connue sous l'appellation pince-crocodile est fixée par pincement sur le guide-fil 20 du cathéter de valve 2. Cette pince 3 est reliée à la cathode d'un stimulateur cardiaque non représenté, situé à l'extérieur du corps C.

Une aiguille non montrée est également plantée dans les tissus sous-cutanés du corps C du patient à opérer. Sur cette aiguille est fixé un fil métallique 4.

Une pince 5 de type crocodile est fixée également par pincement sur le fil métallique 4.

Cette pince 5 est reliée à l'anode du stimulateur cardiaque extérieur au corps.

Ainsi, lorsque la valve artificielle est au niveau de la valve aortique native à remplacer, le chirurgien réalise préalablement à la mise en place proprement dite de la valve artificielle, c'est-à-dire au gonflement du ballon et donc l'expansion du stent auquel est fixée la valve, une stimulation ventriculaire rapide du ventricule gauche.

Pour cela, un signal électrique est délivré entre la cathode et l'anode par le biais des pinces 3, 5, le ballon jouant le rôle d'isolant électrique entre ces deux électrodes.

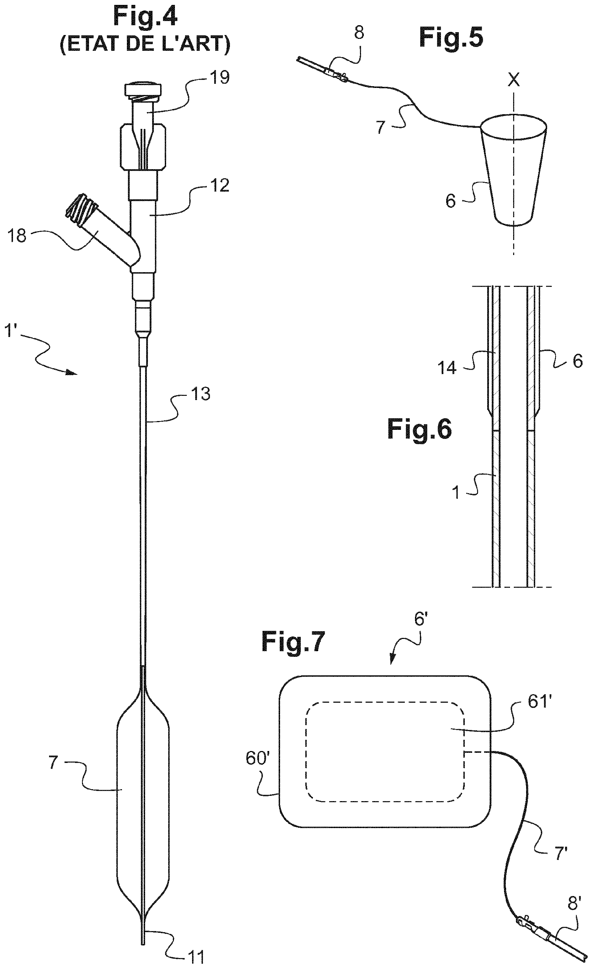

La figure 4 illustre un cathéter de délivrance 1' qui peut être introduit directement dans l'artère d'un patient sans nécessité d'un introducteur. Plus précisément, le cathéter 1' comprend un embout 12 prolongé par une gaine d'introduction 13. L'embout 12 comprend une connexion 18 pour le gonflage/dégonflage d'un ballonnet 7 à l'extrémité distale 11 qui permet d'expandre une valve prothétique non représentée.

Confronté à de nombreuses opérations de remplacement de valve aortique par voie fémorale telle qu'elle vient d'être brièvement décrite, et en particulier à la mise en place précise et délicate de l'aiguille sous-cutanée supplémentaire, ainsi que la mise en place et le maintien des pinces de connexion, de type crocodile sur deux supports éloignés, l'inventeur de la présente invention a déjà pensé à intégrer le fil métallique 4 directement dans un introducteur 1 ou dans un cathéter de délivrance 1'. Cette solution est décrite et revendiquée dans la demande de brevet PCT/EP2016/057385.

Si cette solution amène de nombreux avantages par rapport à la technique selon l'état de l'art, elle présente néanmoins un inconvénient important qui est de nécessiter la réalisation d'introducteurs ou cathéters de délivrance spécifiques.

Aussi, l'inventeur a tout d'abord pensé à intégrer la fonction du fil métallique non pas dans un introducteur ou dans un cathéter de délivrance spécifique mais à réaliser un manchon conducteur électriquement 6 qui est adapté pour venir s'emmancher directement autour d'un introducteur 1 ou cathéter de délivrance 1' existant.

Un tel manchon 6 selon l'invention est représenté en figure 5: il se présente sous la forme d'un tube dont au moins une partie de sa périphérie extérieure est électriquement conductrice.

Cette partie conductrice est reliée à une connexion électrique 8 par le biais d'un fil d'alimentation électrique 7.

Le manchon 6 peut être de forme cylindrique ou en tronc de cône. Sa forme vient épouser au plus près celle de la forme extérieure de la gaine d'introduction 13 d'un introducteur 1 ou d'un cathéter 1'.

Comme visible sur la figure 6, l'épaisseur de ce manchon 6, typiquement de l'ordre du millimètre ou inférieure, ne rajoute qu'une faible surépaisseur à la gaine 13 et donc ne gêne pas la progression celle-ci lors de son introduction dans une artère A.

Le manchon 6 peut être réalisé sous la forme d'une pièce monobloc entièrement conductrice ou sous la forme d'une pièce revêtue d'un revêtement conducteur électrique. En tant que matériau électriquement conducteur, on peut choisir avantageusement du carbone.

Selon une variante avantageuse, on peut concevoir le manchon 6 afin qu'il soit élastique et qu'il puisse ainsi s'adapter à toute taille d'introducteur ou de cathéter de délivrance existant.

En pratique, un chirurgien ou médecin interventionniste souhaitant procéder à une opération de remplacement de valve stimulation cardiaque avec stimulation cardiaque concomitante à la mise en place de la valve prothétique, commence par mettre en place le manchon conducteur 6 autour de l'introducteur 1 ou du cathéter de délivrance 1'. La mise en place du manchon conducteur étant très simple et aisée, elle peut être faite par une assistante ou infirmière sans qu'elle n'ait besoin d'adopter une technique particulière.

La mise en place est atteinte une fois que le manchon 6 est emmanché et agencé autour de la gaine d'introduction 13 pour qu'il vienne toucher soit une zone sous cutanée du patient soit la paroi de l'artère aortique du patient.

Une fois cette mise en place effectuée, l'introduction de l'introducteur 1 ou du cathéter de délivrance 1' avec le manchon 6 autour, peut être faite comme usuellement par le chirurgien.

Une fois le positionnement de l'introducteur 1 ou du cathéter 1' dans l'artère fémorale achevé, la connexion électrique 8 peut être connectée directement à l'anode d'un stimulateur cardiaque externe.

Usuellement, une pince, comme la pince-crocodile 3 représentée en figure 3 peut à son tour être est fixée par pincement sur le guide-fil d'un introducteur 1 ou d'un cathéter de valve 1'. Cette pince est reliée à la cathode du stimulateur cardiaque non représenté, situé à l'extérieur du corps C.

Ainsi, la stimulation cardiaque temporaire pour réaliser la sidération cardiaque souhaitée, peut avoir lieu entre la cathode reliée électriquement au guide-fil et l'anode reliée électriquement au manchon 6 selon l'invention.

L'inventeur a également pensé à intégrer la fonction du fil métallique non pas dans un introducteur ou dans un cathéter de délivrance spécifique mais à utiliser en lieu et place une électrode transcutanée 6'.

Une telle électrode 6' selon l'invention est représentée en figure 7: elle comprend une partie adhésive 60' destinée à être collée sur la peau du corps humain dans lequel la gaine est introduite, et une partie 61' en matériau électrique conducteur adaptée pour délivrer un courant de manière transcutanée.

La partie en matériau conducteur 61' comprend en outre une connexion électrique dont un fil d'alimentation électrique 7' solidaire d'un connecteur 8 pour réaliser la connexion avec une électrode d'un stimulateur cardiaque externe.

En pratique, un chirurgien ou médecin interventionniste souhaitant procéder à une opération de remplacement de valve stimulation cardiaque avec stimulation cardiaque concomitante à la mise en place de la valve prothétique, commence par le collage de l'électrode transcutanée 6 sur la peau du patient. La mise en place de cette électrode peut être faite par exemple dans une zone en regard du cœur.La mise en place étant très simple et aisée, elle peut être faite par une assistante ou infirmière sans qu'elle n'ait besoin d'adopter une technique particulière.

Une fois cette mise en place effectuée, l'introduction de l'introducteur 1 ou du cathéter de délivrance 1' peut être faite comme usuellement par le chirurgien.

Une fois le positionnement de l'introducteur 1 ou du cathéter 1' dans l'artère fémorale achevé, la connexion électrique 8 peut être connectée directement à l'anode d'un stimulateur cardiaque externe.

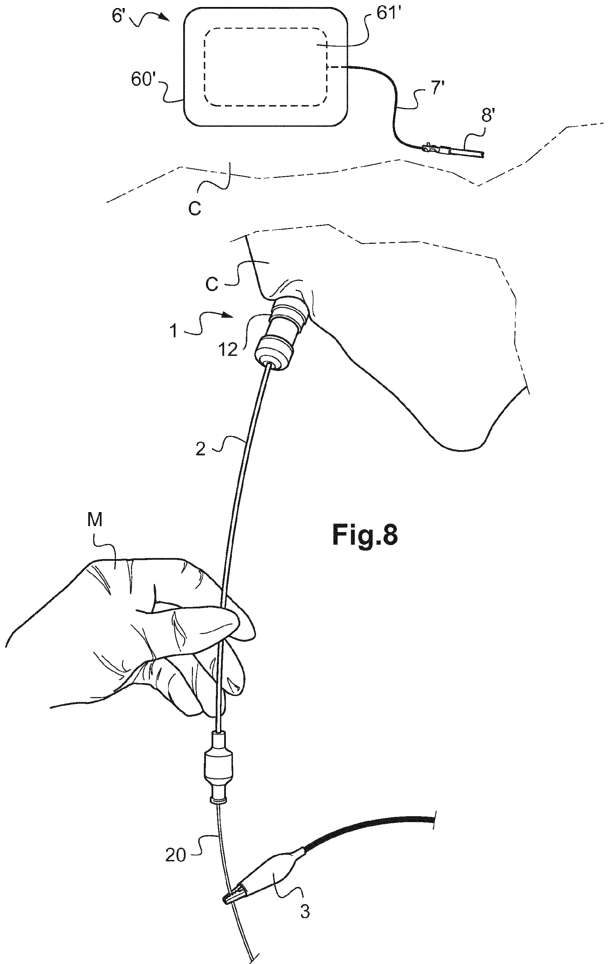

Usuellement, une pince, comme une pince-crocodile 3 peut à son tour être est fixée par pincement sur le guide-fil d'un introducteur 1 ou d'un cathéter de valve 1'. Cette pince est reliée à la cathode du stimulateur cardiaque non représenté, situé à l'extérieur du corps C. Une telle configuration est illustrée en figure 8.

Ainsi, la stimulation cardiaque temporaire pour réaliser la sidération cardiaque souhaitée, peut avoir lieu entre la cathode reliée électriquement au guide-fil et l'anode reliée électriquement à l'électrode transcutanée 6' selon l'invention.

Aussi, l'inventeur a pensé à intégrer la fonction du fil métallique non pas dans un introducteur ou dans un cathéter de délivrance spécifique mais dans un guide-fil bipolaire 6" à isolation électrique sur sa portion centrale, le guide-fil étant toujours destiné à être introduit dans la gaine tubulaire 13 de l'introducteur.

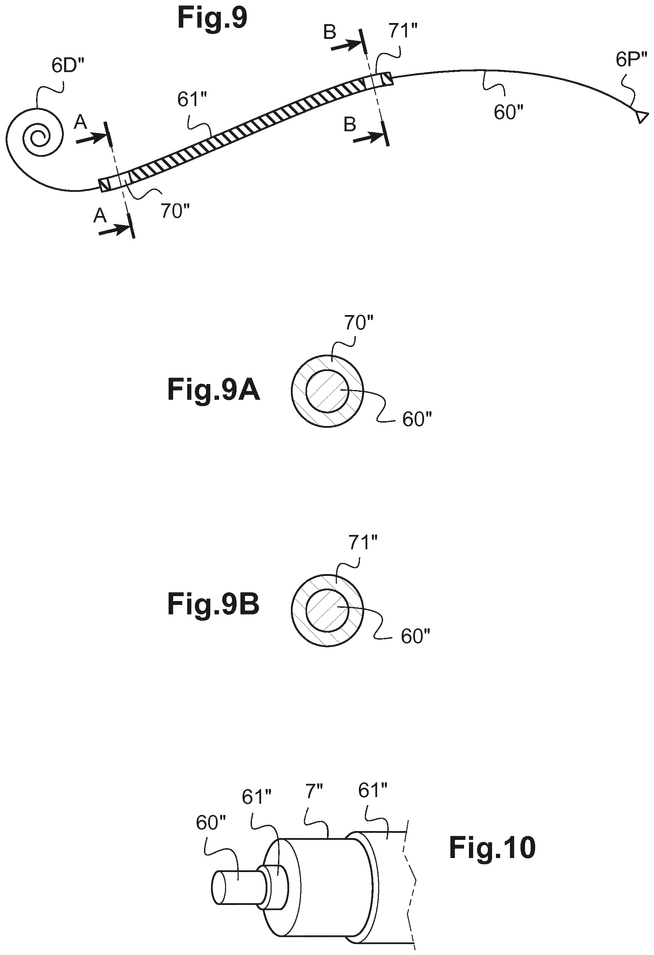

Plus précisément, comme illustré aux figures 9 à 10, le guide-fil bipolaire 6" comprend tout d'abord une âme métallique 60" revêtue d'une gaine électriquement isolante 61" sur une portion centrale entre l'extrémité proximale 6P" et l'extrémité distale 6D" du guide-fil.

L'extrémité distale 6D" de l'âme métallique est destinée à venir en contact avec la paroi du ventricule gauche du cœur d'un patient.

Dans le mode de réalisation illustré, cette extrémité distale 6D" est une portion plus souple que le reste du guide-fil, sa souplesse lui permettant de s'enrouler sur elle-même lorsqu'elle vient en contact avec la paroi du ventricule gauche du cœur d'un patient. On s'assure ainsi d'une mise en contact à la fois sûre et non impactante, c'est-à-dire qui ne risque pas de venir percer la paroi du ventricule, contrairement aux sondes aux sondes de stimulation électro-systolique selon l'état de l'art avec lesquelles le contact est quasi-ponctuel avec la paroi, ce qui induit un risque de tamponnade.

L'âme métallique 60" est non isolée électriquement sur le reste de la longueur du guide fil.

A l'intérieur de la gaine électriquement isolante 61" est noyée une couche métallique 7" à l'exception de ses portions distale 70" et proximale 71".

Ainsi, la portion distale 70" est apparente sur toute la périphérie extérieure de la gaine isolante 71" de sorte à être en contact avec le tissu sous-cutané du corps ou avec l'artère. La portion proximale 71" est apparente sur toute la périphérie extérieure de la gaine isolante de sorte à être accessible depuis l'extérieur du corps C lorsque le guide-fil est introduit dans la gaine d'introduction 13.

Par cette constitution du guide-fil bipolaire 6", la portion proximale 71" de l'élément conducteur intégré 7" sert de connexion à une électrode d'un stimulateur cardiaque externe au corps, tandis que l'âme métallique 60" du guide-fil bipolaire sert de connexion à l'autre électrode du stimulateur cardiaque externe.

En pratique, un chirurgien ou médecin interventionniste souhaitant procéder à une opération de remplacement de valve stimulation cardiaque avec stimulation cardiaque concomitante à la mise en place de la valve prothétique, commence par l'introduction de l'introducteur 1 ou du cathéter de délivrance 1' peut être faite comme usuellement par le chirurgien.

Le guide-fil bipolaire 6" selon l'invention est alors introduit dans la gaine d'introduction 13 de l'introducteur 1 ou du cathéter de délivrance 1'.

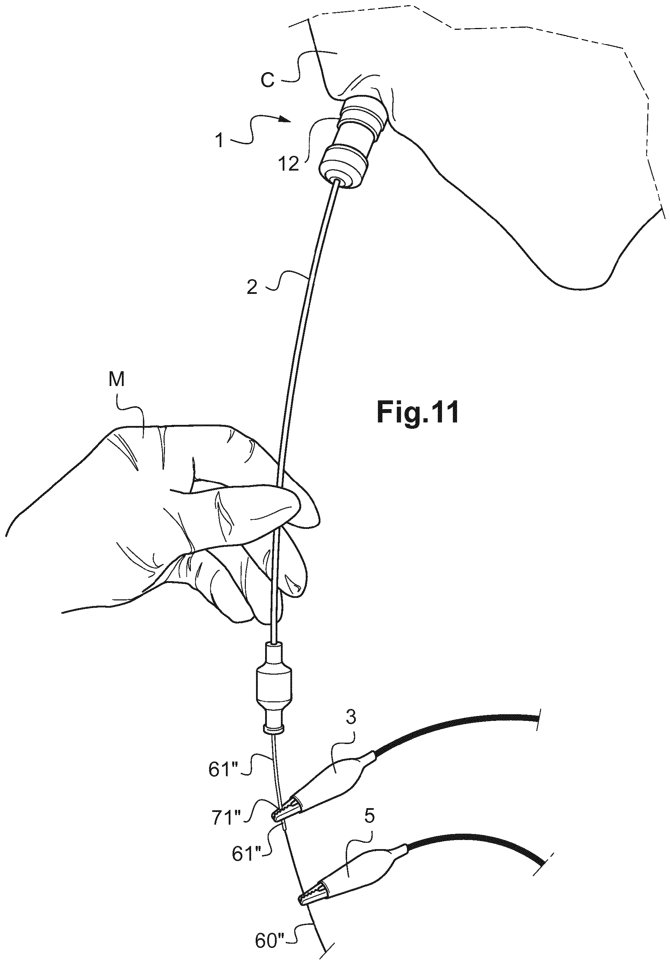

Une fois ces mises en places achevées, la portion proximale 71" de l'élément conducteur 7" peut être connectée directement à l'anode d'un stimulateur cardiaque externe. De manière alternative, comme illustré en figure 11, une pince 5, comme une pince-crocodile 3 peut être fixée par pincement sur la portion proximale conductrice 71". Cette pince 3 est reliée à l'anode du stimulateur cardiaque non représenté, situé à l'extérieur du corps C.

Usuellement, une pince, comme une pince-crocodile 5 peut à son tour être fixée par pincement sur l'âme métallique 60" du guide-fil 6". Cette pince 5 est reliée à la cathode du stimulateur cardiaque non représenté, situé à l'extérieur du corps C. Une telle configuration est illustrée en figure 11.

Ainsi, la stimulation cardiaque temporaire pour réaliser la sidération cardiaque souhaitée, peut avoir lieu entre la cathode reliée électriquement à l'âme métallique 60" du guide-fil 6" selon l'invention et l'anode reliée électriquement la portion distale 71" de l'élément conducteur 7" du guide-fil 6".

L'invention permet de conserver tous les avantages inhérents à l'invention selon la demande PCT/EP2016/057385 pour le remplacement d'une valve cardiaque par voie percutanée avec en outre l'avantage de pouvoir être mise en œuvre sur tout introducteur ou cathéter de délivrance existant, puisqu'au choix:
- seul un manchon conducteur 6 doit être emmanché autour dudit introducteur ou cathéter existant et une connexion électrique sur le guide-fil doit être effectuée, au préalable de leur introduction dans l'artère du patient à opérer ; ou
- seule une électrode transcutanée 6' est à coller sur la peau d'un patient en vue de la sidération cardiaque et une connexion est à faire sur le guide-fil lors de l'opération de remplacement ;
- seules deux connexions électriques sont à faire sur le guide-fil 6" lors de l'opération de remplacement.

L'invention n'est pas limitée aux exemples qui viennent d'être décrits ; on peut notamment combiner entre elles des caractéristiques des exemples illustrés au sein de variantes non illustrées.

D'autres variantes et améliorations peuvent être prévues sans pour autant sortir du cadre de l'invention.

### Références citées

[1]: « Registry of Transcatheter Aortic-Valve Implantation in High-Risk Patients », Gilard et al ; the New England Journal of Medicine : pp 1705-1715
[2]: « Left Ventricular Guidewire Pacing to Simplify Aortic Balloon Valvuloplasty », Susanne Navarini et al; Catheterization and Cardiovascular Interventions 73: pp 426-427 (2009)
[3]: « A novel Approach for Transcoronary Pacing un a Porcine Model », Roland Prodzinsky et al ; Journal of Invasive Cardiology 24(9) : pp 451-455 (2012)
[4]: « Optimizing of Transcoronary Pacing in a Porcine Model », Konstantin M. Heinroth, et al, Journal of Invasive Cardiology 21, pp 634-638 (2009)

## Revendications

1. Ensemble de remplacement d'une valve cardiaque par voie percutanée, comprenant:
- un dispositif formant un introducteur (1) comprenant au moins une gaine tubulaire (13) d'introduction, destinée à être introduite dans une artère d'un corps humain (C) et à laisser passer un dispositif d'intervention chirurgicale, tel qu'un cathéter de délivrance , **caractérisé en ce qu'**il comprend:
- un manchon (6) adapté pour être emmanché autour de la gaine d'introduction, le manchon étant en matériau électrique conducteur sur au moins une partie de sa périphérie extérieure de sorte que lorsque la gaine avec le manchon emmanché autour est introduite dans l'artère d'un corps humain, la périphérie conductrice du manchon est en contact avec le tissu sous-cutané du corps ou avec l'artère, le manchon comprenant en outre une connexion électrique (7, 8) à une électrode d'un stimulateur cardiaque externe au corps;
- au moins un guide-fil (20) destiné à être introduit dans la gaine tubulaire (13) de l'introducteur pour l'avancement d'une valve artificielle destinée à remplacer la valve cardiaque, le guide-fil (20) comprenant une partie métallique servant en outre de connexion à l'autre électrode du stimulateur cardiaque externe.

2. Ensemble de remplacement d'une valve cardiaque par voie percutanée, comprenant:
- un dispositif formant un cathéter de délivrance de valve (1') comprenant au moins une gaine tubulaire (13) d'introduction, destinée à être introduit dans une artère d'un corps humain, **caractérisé en ce qu'**il comprend:
- un manchon (6) adapté pour être emmanché autour de la gaine d'introduction, le manchon étant en matériau électrique conducteur sur au moins une partie de sa périphérie extérieure de sorte que lorsque la gaine avec le manchon emmanché autour est introduit dans l'artère d'un corps humain, la périphérie conductrice du manchon est en contact avec le tissu sous-cutané du corps ou avec l'artère, le manchon comprenant en outre une connexion électrique (7, 8) à une électrode d'un stimulateur cardiaque externe au corps;
- au moins un guide-fil (20) destiné à être introduit dans la gaine tubulaire (13) du cathéter de délivrance pour l'avancement d'une valve artificielle destinée à remplacer la valve cardiaque, le guide-fil comprenant au moins une partie métallique (20) servant en outre de connexion à l'autre électrode du stimulateur cardiaque externe.

3. Ensemble selon la revendication 1 ou 2, l'électrode du stimulateur cardiaque connectée au manchon conducteur électrique (6) emmanché autour de la gaine d'introduction de l'introducteur (1) ou du cathéter de délivrance étant l'anode tandis que celle connectée à la partie métallique du guide-fil (20) est la cathode.

4. Ensemble selon l'une des revendications 1 à 3, dans lequel le manchon électriquement conducteur est constitué d'une pièce monobloc en matériau conducteur, tel que du carbone.

5. Ensemble selon revendication 4, le manchon étant constitué d'une gaine comprenant sur sa périphérie extérieure un revêtement conducteur électrique (4), tel qu'un revêtement en carbone.

6. Ensemble selon la revendication 4 ou 5, le manchon étant élastique de sorte à pouvoir s'emmancher sur des gaines d'introducteurs ou des cathéters de délivrance de différents diamètres, typiquement des diamètres externes compris entre 1,67 et 8 mm.

7. Ensemble de remplacement d'une valve cardiaque par voie percutanée, comprenant:
- un dispositif formant un introducteur (1) comprenant au moins une gaine tubulaire (13) d'introduction, destinée à être introduite dans une artère d'un corps humain (C) et à laisser passer un dispositif d'intervention chirurgicale, tel qu'un cathéter de délivrance, **caractérisé en ce qu'**il comprend:
- une électrode transcutanée (6'), comprenant une partie adhésive (60') destinée à être collée sur la peau du corps humain dans lequel la gaine est introduite, et une partie (61) en matériau électrique conducteur de sorte que lorsque la partie adhésive est collée à la peau, la partie conductrice peut délivrer un courant de manière transcutanée, la partie conductrice comprenant en outre une connexion électrique (7, 8) à une électrode d'un stimulateur cardiaque externe au corps;
- au moins un guide-fil (20) destiné à être introduit dans la gaine tubulaire (13) de l'introducteur pour l'avancement d'une valve artificielle destinée à remplacer la valve cardiaque, le guide-fil (20) comprenant une partie métallique servant en outre de connexion à l'autre électrode du stimulateur cardiaque externe.

8. Ensemble de remplacement d'une valve cardiaque par voie percutanée, comprenant:
- un dispositif formant un cathéter de délivrance de valve (1') comprenant au moins une gaine tubulaire (13) d'introduction, destinée à être introduit dans une artère d'un corps humain, **caractérisé en ce qu'**il comprend:
- une électrode transcutanée (6'), comprenant une partie adhésive (60') destinée à être collée sur la peau du corps humain dans lequel la gaine est introduite, et une partie (61) en matériau électrique conducteur de sorte que lorsque la partie adhésive est collée à la peau, la partie conductrice peut délivrer un courant de manière transcutanée, la partie conductrice comprenant en outre une connexion électrique (7, 8) à une électrode d'un stimulateur cardiaque externe au corps;
- au moins un guide-fil (20) destiné à être introduit dans la gaine tubulaire (13) du cathéter de délivrance pour l'avancement d'une valve artificielle destinée à remplacer la valve cardiaque, le guide-fil comprenant au moins une partie métallique (20) servant en outre de connexion à l'autre électrode du stimulateur cardiaque externe.

9. Ensemble selon la revendication 7 ou 8, l'électrode du stimulateur cardiaque connectée à l'électrode transcutanée (6') étant l'anode tandis que celle connectée à la partie métallique du guide-fil (20) est la cathode.

10. Ensemble de remplacement d'une valve cardiaque par voie percutanée, comprenant:
- un dispositif formant un introducteur (1) comprenant au moins une gaine tubulaire (13) d'introduction, destinée à être introduite dans une artère d'un corps humain (C) et à laisser passer un dispositif d'intervention chirurgicale, tel qu'un cathéter de délivrance, **caractérisé en ce qu'**il comprend:
- au moins un guide-fil (6"), dit guide-fil bipolaire, destiné à être introduit dans la gaine tubulaire (13) de l'introducteur pour l'avancement d'une valve artificielle destinée à remplacer la valve cardiaque, le guide-fil (6") comprenant une âme métallique (60") revêtue d'une gaine électriquement isolante (61") sur une portion centrale entre l'extrémité proximale (6P) et l'extrémité distale (6D) du guide-fil, l'âme métallique étant non isolée électriquement sur le reste de la longueur du guide fil, la gaine électriquement isolante intégrant un élément conducteur électrique (7") dont une portion distale (70") est apparente sur une au moins une partie de la périphérie extérieure de la gaine isolante de sorte à être en contact avec le tissu sous-cutané du corps ou avec l'artère et dont une portion proximale (71") est apparente sur une au moins une partie de la périphérie extérieure de la gaine isolante de sorte à être accessible depuis l'extérieur du corps (C) lorsque le guide-fil est introduit dans la gaine d'introduction (13), la portion proximale (71") servant de connexion à une électrode d'un stimulateur cardiaque externe au corps, tandis que l'âme métallique (60") du guide-fil bipolaire sert de connexion à l'autre électrode du stimulateur cardiaque externe.

11. Ensemble de remplacement d'une valve cardiaque par voie percutanée, comprenant:
- un dispositif formant un cathéter de délivrance de valve (1') comprenant au moins une gaine tubulaire (13) d'introduction, destinée à être introduit dans une artère d'un corps humain, **caractérisé en ce qu'**il comprend:
- au moins un guide-fil (6"), dit guide-fil bipolaire, destiné à être introduit dans la gaine tubulaire (13) d'introduction pour l'avancement d'une valve artificielle destinée à remplacer la valve cardiaque, le guide-fil (6") comprenant une âme métallique (60") revêtue d'une gaine électriquement isolante (61) sur une portion centrale entre l'extrémité proximale (6P") et l'extrémité distale (6D") du guide-fil, l'âme métallique étant non isolée électriquement sur le reste de la longueur du guide fil, la gaine électriquement isolante intégrant un élément conducteur électrique (7") dont une portion distale (70") est apparente sur une au moins une partie de la périphérie extérieure de la gaine isolante de sorte à être en contact avec le tissu sous-cutané du corps ou avec l'artère et dont une portion proximale (71") est apparente sur une au moins une partie de la périphérie extérieure de la gaine isolante de sorte à être accessible depuis l'extérieur du corps (C) lorsque le guide-fil est introduit dans la gaine d'introduction (13), la portion proximale (71") servant de connexion à une électrode d'un stimulateur cardiaque externe au corps, tandis que l'âme métallique (60") du guide-fil bipolaire sert de connexion à l'autre électrode du stimulateur cardiaque externe.

12. Ensemble selon la revendication 10 ou 11, l'électrode du stimulateur cardiaque connectée à l'élément conducteur électrique (7") intégré dans la gaine du guide-fil (6") étant l'anode, tandis que celle connectée à l'âme métallique (60") du guide-fil (6") est la cathode.

13. Ensemble selon l'une des revendications 10 à 12, l'élément conducteur métallique (7") étant une couche métallique noyée dans la gaine isolante (61") à l'exception de ses portions apparentes, distale (70") et proximale (71").

14. Ensemble selon l'une des revendications 10 à 13, l'extrémité distale (6D") non isolée électriquement, de l'âme métallique (60") du guide-fil, destinée à venir en contact avec la paroi du ventricule gauche du cœur d'un patient, étant une portion plus souple que le reste du guide-fil, de préférence la portion plus souple (6D") étant adaptée pour s'enrouler sur elle-même lorsqu'elle vient en contact avec la paroi du ventricule gauche du cœur d'un patient.

15. Ensemble selon l'une des revendications 10 à 14, la portion distale (71") de l'élément conducteur étant reliée à une connexion électrique, destinée elle-même à être reliée à une électrode d'un stimulateur cardiaque externe au corps.

## Patentansprüche

1. Anordnung zum Ersetzen einer Herzklappe auf perkutanem Weg, umfassend:
- eine einen Einführer (1) bildende Vorrichtung, die mindestens eine röhrenförmige Einführungshülle (13) umfasst, die dazu bestimmt ist, in eine Arterie eines menschlichen Körpers (C) eingeführt zu werden und eine chirurgische Eingriffsvorrichtung wie einen Zuführkatheter durchgehen zu lassen, **dadurch gekennzeichnet, dass** sie umfasst:
- eine Hülse (6), die geeignet ist, um die Einführungshülle aufgeschoben zu werden, wobei die Hülse über mindestens einen Teil ihres Außenumfangs aus elektrisch leitfähigem Material ist, so dass, wenn die Hülle mit der um sie aufgeschobenen Hülse in die Arterie eines menschlichen Körpers eingeführt ist, der leitfähige Umfang der Hülse mit dem subkutanen Gewebe des Körpers oder mit der Arterie in Kontakt ist, wobei die Hülse ferner einen elektrischen Anschluss (7, 8) an eine Elektrode eines körperexternen Herzschrittmachers umfasst;
- mindestens einen Führungsdraht (20), der dazu bestimmt ist, zum Vorschieben einer zum Ersetzen der Herzklappe bestimmten künstlichen Klappe in die röhrenförmige Hülle (13) eingeführt zu werden, wobei der Führungsdraht (20) einen metallischen Teil umfasst, der ferner als Anschluss an die andere Elektrode des externen Herzschrittmachers dient.

2. Anordnung zum Ersetzen einer Herzklappe auf perkutanem Weg, umfassend:
- eine einen Klappenzuführungskatheter (1') bildende Vorrichtung, die mindestens eine röhrenförmige Einführungshülle (13) umfasst, die dazu bestimmt ist, in eine Arterie eines menschlichen Körpers eingeführt zu werden, **dadurch gekennzeichnet, dass** sie umfasst:
- eine Hülse (6), die geeignet ist, um die Einführungshülle aufgeschoben zu werden, wobei die Hülse über mindestens einen Teil ihres Außenumfangs aus elektrisch leitfähigem Material ist, so dass, wenn die Hülle mit der um sie aufgeschobenen Hülse in die Arterie eines menschlichen Körpers eingeführt ist, der leitfähige Umfang der Hülse mit dem subkutanen Gewebe des Körpers oder mit der Arterie in Kontakt ist, wobei die Hülse ferner einen elektrischen Anschluss (7, 8) an eine Elektrode eines körperexternen Herzschrittmachers umfasst;
- mindestens einen Führungsdraht (20), der dazu bestimmt ist, zum Vorschieben einer zum Ersetzen der Herzklappe bestimmten künstlichen Klappe in die röhrenförmige Hülle (13) eingeführt zu werden, wobei der Führungsdraht einen metallischen Teil (20) umfasst, der ferner als Anschluss an die andere Elektrode des externen Herzschrittmachers dient.

3. Anordnung nach Anspruch 1 oder 2, wobei die Elektrode des Herzschrittmachers, die an die elektrisch leitfähige Hülse (6) angeschlossen ist, die um die Einführungshülle des Einführers (1) oder den Zuführungskatheter aufgeschoben ist, die Anode ist, während die an den metallischen Teil des Führungsdrahts (20) angeschlossene die Kathode ist.

4. Anordnung nach einem der Ansprüche 1 bis 3, wobei die elektrisch leitfähige Hülse aus einem einstückigen Teil aus leitfähigem Material wie Kohlenstoff besteht.

5. Anordnung nach Anspruch 4, wobei die Hülse aus einer Hülle besteht, die auf ihrem Außenumfang einen elektrisch leitfähigen Überzug (4) wie einen Kohlenstoffüberzug umfasst.

6. Anordnung nach Anspruch 4 oder 5, wobei die Hülse elastisch ist, so dass sie auf Hüllen von Einführern oder Zuführungskathetern mit unterschiedlichen Durchmessern, typischerweise Außendurchmessern zwischen 1,67 und 8 mm, aufgeschoben werden kann.

7. Anordnung zum Ersetzen einer Herzklappe auf perkutanem Weg, umfassend:
- eine einen Einführer (1) bildende Vorrichtung, die mindestens eine röhrenförmige Einführungshülle (13) umfasst, die dazu bestimmt ist, in eine Arterie eines menschlichen Körpers (C) eingeführt zu werden und eine chirurgische Eingriffsvorrichtung wie einen Zuführkatheter durchgehen zu lassen, **dadurch gekennzeichnet, dass** sie umfasst:
- eine transkutane Elektrode (6'), umfassend einen haftenden Teil (60'), der dazu bestimmt ist, auf die Haut des menschlichen Körpers geklebt zu werden, in den die Hülle eingeführt wird, und einen Teil (61) aus elektrisch leitfähigem Material, so dass, wenn der haftende Teil auf die Haut geklebt ist, der leitfähige Teil transkutan einen Strom abgeben kann, wobei der leitfähige Teil ferner einen elektrischen Anschluss (7, 8) an eine Elektrode eines körperexternen Herzschrittmachers umfasst;
- mindestens einen Führungsdraht (20), der dazu bestimmt ist, zum Vorschieben einer zum Ersetzen der Herzklappe bestimmten künstlichen Klappe in die röhrenförmige Hülle (13) eingeführt zu werden, wobei der Führungsdraht (20) einen metallischen Teil umfasst, der ferner als Anschluss an die andere Elektrode des externen Herzschrittmachers dient.

8. Anordnung zum Ersetzen einer Herzklappe auf perkutanem Weg, umfassend:
- eine einen Klappenzuführungskatheter (1') bildende Vorrichtung, die mindestens eine röhrenförmige Einführungshülle (13) umfasst, die dazu bestimmt ist, in eine Arterie eines menschlichen Körpers eingeführt zu werden, **dadurch gekennzeichnet, dass** sie umfasst:
- eine transkutane Elektrode (6'), umfassend einen haftenden Teil (60'), der dazu bestimmt ist, auf die Haut des menschlichen Körpers geklebt zu werden, in den die Hülle eingeführt wird, und einen Teil (61) aus elektrisch leitfähigem Material, so dass, wenn der haftende Teil auf die Haut geklebt ist, der leitfähige Teil transkutan einen Strom abgeben kann, wobei der leitfähige Teil ferner einen elektrischen Anschluss (7, 8) an eine Elektrode eines körperexternen Herzschrittmachers umfasst;
- mindestens einen Führungsdraht (20), der dazu bestimmt ist, zum Vorschieben einer zum Ersetzen der Herzklappe bestimmten künstlichen Klappe in die röhrenförmige Hülle (13) eingeführt zu werden, wobei der Führungsdraht einen metallischen Teil (20) umfasst, der ferner als Anschluss an die andere Elektrode des externen Herzschrittmachers dient.

9. Anordnung nach Anspruch 7 oder 8, wobei die an die transkutane Elektrode (6') angeschlossene Elektrode des Herzschrittmachers die Anode ist, während die an den metallischen Teil des Führungsdrahts (20) angeschlossene die Kathode ist.

10. Anordnung zum Ersetzen einer Herzklappe auf perkutanem Weg, umfassend:
- eine einen Einführer (1) bildende Vorrichtung, die mindestens eine röhrenförmige Einführungshülle (13) umfasst, die dazu bestimmt ist, in eine Arterie eines menschlichen Körpers (C) eingeführt zu werden und eine chirurgische Eingriffsvorrichtung wie einen Zuführkatheter durchgehen zu lassen, **dadurch gekennzeichnet, dass** sie umfasst:
- mindestens einen Führungsdraht (6"), bipolarer Führungsdraht genannt, der dazu bestimmt ist, zum Vorschieben einer zum Ersetzen der Herzklappe bestimmten künstlichen Klappe in die röhrenförmige Hülle (13) des Einführers eingeführt zu werden, wobei der Führungsdraht (6") eine metallische Seele (60") umfasst, die über einen zentralen Abschnitt zwischen dem proximalen Ende (6P) und dem distalen Ende (6D) des Führungsdrahts mit einer elektrisch isolierenden Hülle (61") überzogen ist, wobei die metallische Seele über den Rest der Länge des Führungsdrahts nicht elektrisch isoliert ist, wobei die elektrisch isolierende Hülle ein elektrisch leitfähiges Element (7") beinhaltet, von dem ein distaler Abschnitt (70") über einen mindestens einen Teil des Außenumfangs der isolierenden Hülle sichtbar ist, so dass er mit dem subkutanen Gewebe oder mit der Arterie in Kontakt ist, und von dem ein proximaler Abschnitt (71") über einen mindestens einen Teil des Außenumfangs der isolierenden Hülle sichtbar ist, so dass er von außerhalb des Körpers (C) zugänglich ist, wenn der Führungsdraht in die Einführungshülle (13) eingeführt ist, wobei der proximale Abschnitt (71") als Anschluss an eine Elektrode eines körperexternen Herzschrittmachers dient, während die metallische Seele (60") des bipolaren Führungsdrahts als Anschluss an die andere Elektrode des externen Herzschrittmachers dient.

11. Anordnung zum Ersetzen einer Herzklappe auf perkutanem Weg, umfassend:
- eine einen Klappenzuführungskatheter (1') bildende Vorrichtung, die mindestens eine röhrenförmige Einführungshülle (13) umfasst, die dazu bestimmt ist, in eine Arterie eines menschlichen Körpers eingeführt zu werden, **dadurch gekennzeichnet, dass** sie umfasst:
- mindestens einen Führungsdraht (6"), bipolarer Führungsdraht genannt, der dazu bestimmt ist, zum Vorschieben einer zum Ersetzen der Herzklappe bestimmten künstlichen Klappe in die röhrenförmige Einführungshülle (13) eingeführt zu werden, wobei der Führungsdraht (6") eine metallische Seele (60") umfasst, die über einen zentralen Abschnitt zwischen dem proximalen Ende (6P") und dem distalen Ende (6D") des Führungsdrahts mit einer elektrisch isolierenden Hülle (61) überzogen ist, wobei die metallische Seele über den Rest der Länge des Führungsdrahts nicht elektrisch isoliert ist, wobei die elektrisch isolierende Hülle ein elektrisch leitfähiges Element (7") beinhaltet, von dem ein distaler Abschnitt (70") über einen mindestens einen Teil des Außenumfangs der isolierenden Hülle sichtbar ist, so dass er mit dem subkutanen Gewebe oder mit der Arterie in Kontakt ist, und von dem ein proximaler Abschnitt (71") über einen mindestens einen Teil des Außenumfangs der isolierenden Hülle sichtbar ist, so dass er von außerhalb des Körpers (C) zugänglich ist, wenn der Führungsdraht in die Einführungshülle (13) eingeführt ist, wobei der proximale Abschnitt (71") als Anschluss an eine Elektrode eines körperexternen Herzschrittmachers dient, während die metallische Seele (60") des bipolaren Führungsdrahts als Anschluss an die andere Elektrode des externen Herzschrittmachers dient.

12. Anordnung nach Anspruch 10 oder 11, wobei die Elektrode des Herzschrittmachers, die an das in die Hülle des Führungsdrahts (6") integrierte elektrisch leitfähige Element (7") angeschlossen ist, die Anode ist, während die an die metallische Seele (60") des Führungsdrahts (6") angeschlossene die Kathode ist.

13. Anordnung nach einem der Ansprüche 10 bis 12, wobei das metallische leitfähige Element (7") eine metallische Schicht ist, die in die isolierende Hülle (61") eingebettet ist, mit Ausnahme von deren sichtbaren distalen (70") und proximalen (71") Abschnitten.

14. Anordnung nach einem der Ansprüche 10 bis 13, wobei das elektrisch nicht isolierte distale Ende (6D") der metallischen Seele (60") des Führungsdrahts, das dazu bestimmt ist, mit der Wand des linken Herzventrikels eines Patienten in Kontakt zu gelangen, ein Abschnitt ist, der flexibler als der Rest des Führungsdrahts ist, wobei der flexiblere Abschnitt (6D") bevorzugt geeignet ist, sich aufzurollen, wenn er mit der Wand des linken Herzventrikels eines Patienten in Kontakt gelangt.

15. Anordnung nach einem der Ansprüche 10 bis 14, wobei der distale Abschnitt (71") des leitfähigen Elements mit einem elektrischen Anschluss verbunden ist, der wiederum dazu bestimmt ist, mit einer Elektrode eines körperexternen Herzschrittmachers verbunden zu werden.

## Claims

1. An assembly for replacing a heart valve by the percutaneous route, comprising:
- a device forming an introducer (1) comprising at least one tubular introduction sheath (13), intended to be introduced into an artery of a human body (C) and to allow through a surgical intervention device, such as a delivery catheter;
**characterized in that** it comprises:
- a sleeve (6) adapted to be fitted around the introduction sheath, the sleeve being made of electrically conductive material on at least one part of the outer periphery thereof so that, when the sheath is introduced into the artery of a human body with the sleeve fitted around the sheath, the conductive periphery of the sleeve is in contact with the subcutaneous tissue of the body or with the artery, the sleeve further comprising an electrical connection (7, 8) to an electrode of a cardiac stimulator outside the body;
- at least one guide wire (20) intended to be introduced into the tubular sheath (13) of the introducer in order to advance an artificial valve intended to replace the heart valve, the guide wire (20) comprising a metal part also acting as a connection to the other electrode of the external cardiac stimulator.

2. An assembly for replacing a heart valve by the percutaneous route, comprising:
- a device forming a valve delivery catheter (1') comprising at least one tubular introduction sheath (13), intended to be introduced into an artery of a human body;
**characterized in that** it comprises:
- a sleeve (6) adapted to be fitted around the introduction sheath, the sleeve being made of electrically conductive material on at least one part of the outer periphery thereof so that, when the sheath is introduced into the artery of a human body with the sleeve fitted around the sheath, the conductive periphery of the sleeve is in contact with the subcutaneous tissue of the body or with the artery, the sleeve further comprising an electrical connection (7, 8) to an electrode of a cardiac stimulator outside the body;
- at least one guide wire (20) intended to be introduced into the tubular sheath (13) of the delivery catheter in order to advance an artificial valve intended to replace the heart valve, the guide wire (20) comprising at least one metal part also acting as a connection to the other electrode of the external cardiac stimulator.

3. The assembly as claimed in claim 1 or claim 2, the electrode of the cardiac stimulator connected to the electrically conductive sleeve (6) fitted around the introduction sheath of the introducer (1) or of the delivery catheter being the anode, whereas that which is connected to the metal part of the guide wire (20) is the cathode.

4. The assembly as claimed in any of claims 1 to 3, wherein the electrically conductive sleeve is formed by a one-piece part made of conductive material, such as carbon.

5. The assembly as claimed in claim 4, the sleeve being formed by a sheath comprising an electrically conductive coating (4), such as a carbon coating, on the outer periphery thereof.

6. The assembly as claimed in claim 4 or claim 5, the sleeve being resilient so as to be able to be fitted onto sheaths of introducers or delivery catheters with different diameters, typically with outer diameters between 1.67 and 8 mm.

7. An assembly for replacing a heart valve by the percutaneous route, comprising:
- a device forming an introducer (1) comprising at least one tubular introduction sheath (13), intended to be introduced into an artery of a human body (C) and to allow through a surgical intervention device, such as a delivery catheter;
**characterized in that** it comprises:
- a transcutaneous electrode (6') comprising an adhesive part (60') intended to be adhered to the skin of the human body into which the sheath is introduced, and a part (61) made of electrically conductive material so that, when the adhesive part is adhered to the skin, the conductive part can deliver a current transcutaneously, the conductive part further comprising an electrical connection (7, 8) to an electrode of a cardiac stimulator outside the body;
- at least one guide wire (20) intended to be introduced into the tubular sheath (13) of the introducer in order to advance an artificial valve intended to replace the heart valve, the guide wire (20) comprising a metal part also acting as a connection to the other electrode of the external cardiac stimulator.

8. An assembly for replacing a heart valve by the percutaneous route, comprising:
- a device forming a valve delivery catheter (1') comprising at least one tubular introduction sheath (13), intended to be introduced into an artery of a human body;
**characterized in that** it comprises:
- a transcutaneous electrode (6') comprising an adhesive part (60') intended to be adhered to the skin of the human body into which the sheath is introduced, and a part (61) made of electrically conductive material so that, when the adhesive part is adhered to the skin, the conductive part can deliver a current transcutaneously, the conductive part further comprising an electrical connection (7, 8) to an electrode of a cardiac stimulator outside the body;
- at least one guide wire (20) intended to be introduced into the tubular sheath (13) of the delivery catheter in order to advance an artificial valve intended to replace the heart valve, the guide wire (20) comprising at least one metal part also acting as a connection to the other electrode of the external cardiac stimulator.

9. The assembly as claimed in claim 7 or claim 8, the electrode of the cardiac stimulator connected to the transcutaneous electrode (6') being the anode, whereas that which is connected to the metal part of the guide wire (20) is the cathode.

10. An assembly for replacing a heart valve by the percutaneous route, comprising:
- a device forming an introducer (1) comprising at least one tubular introduction sheath (13), intended to be introduced into an artery of a human body (C) and to allow through a surgical intervention device, such as a delivery catheter;
**characterized in that** it comprises:
- at least one guide wire (6"), called bipolar guide wire, intended to be introduced into the tubular sheath (13) in order to advance an artificial valve intended to replace the heart valve, the guide wire (6") comprising a metal core (60") coated with an electrically isolating sheath (61") on a central portion between the proximal end (6P) and the distal end (6D) of the guide wire, the metal core being non-electrically isolated on the remainder of the length of the guide wire, the electrically isolating sheath integrating an electrically conductive element (7"), for which a distal portion (70") is apparent on at least one part of the outer periphery of the isolating sheath so as to be in contact with the subcutaneous tissue of the body or with the artery and for which a proximal portion (71") is apparent on at least one part of the outer periphery of the isolating sheath so as to be accessible from outside the body (C) when the guide wire is introduced into the introduction sheath (13), with the proximal portion (71") acting as a connection to an electrode of a cardiac stimulator outside the body, whereas the metal core (60") of the bipolar guide wire acts as a connection to the other electrode of the external cardiac stimulator.

11. An assembly for replacing a heart valve by the percutaneous route, comprising:
- a device forming a valve delivery catheter (1') comprising at least one tubular introduction sheath (13), intended to be introduced into an artery of a human body;
**characterized in that** it comprises:
- at least one guide wire (6"), called bipolar guide wire, intended to be introduced into the tubular sheath (13) of the introducer in order to advance an artificial valve intended to replace the heart valve, the guide wire (6") comprising a metal core (60") coated with an electrically isolating sheath (61) on a central portion between the proximal end (6P") and the distal end (6D") of the guide wire, the metal core being non-electrically isolated on the remainder of the length of the guide wire, the electrically isolating sheath integrating an electrically conductive element (7"), for which a distal portion (70") is apparent on at least one part of the outer periphery of the isolating sheath so as to be in contact with the subcutaneous tissue of the body or with the artery and for which a proximal portion (71") is apparent on at least one part of the outer periphery of the isolating sheath so as to be accessible from outside the body (C) when the guide wire is introduced into the introduction sheath (13), with the proximal portion (71") acting as a connection to an electrode of a cardiac stimulator outside the body, whereas the metal core (60") of the bipolar guide wire acts as a connection to the other electrode of the external cardiac stimulator.

12. The assembly as claimed in claim 10 or claim 11, the electrode of the cardiac stimulator connected to the electrically conductive element (7") integrated in the sheath of the guide wire (6") being the anode, whereas that which is connected to the metal core (60") of the guide wire (6") is the cathode.

13. The assembly as claimed in any of claims 10 to 12, the metal conductive element (7") being a metal layer embedded in the isolating sheath (61 "), except for its apparent distal (70") and proximal (71") portions.

14. The assembly as claimed in any of claims 10 to 13, the non-electrically isolated distal end (6D") of the metal core (60") of the guide wire intended to come into contact with the wall of the left ventricle of the heart of a patient being a portion that is more flexible than the remainder of the guide wire, preferably the more flexible portion (6D") being adapted to wrap around itself when it comes into contact with the wall of the left ventricle of the heart of a patient.

15. The assembly as claimed in any of claims 10 to 14, the distal portion (71") of the conductive element being connected to an electrical connection, which is intended to be connected to an electrode of a cardiac stimulator outside the body.
